# EUROPEAN PATENT SPECIFICATION

(11) **EP 2 740 414 B1**
(45) Date of publication and mention of the grant of the patent: **07.02.2018**
(21) Application number: 13195554.4
(22) Date of filing: 03.12.2013
(51) Int. Cl.: A61B 17/04, A61B 17/00, A61B 17/064, A61B 17/06

(54) **Suture anchor delivery device**
Fadenanker-Einbringinstrument
Instrument de distribution d'un ancre de suture

(30) Priority: 06.12.2012 US 201213707229
(43) Date of publication of application: 11.06.2014
(73) Proprietor: Karl Storz SE & Co. KG, 78532 Tuttlingen (DE)
(72) Inventor: Hart, Rickey, Goleta, 93117 (US); Zantop, Thore, 94315 Straubing (DE)
(74) Representative: Witte, Weller & Partner Patentanwälte mbB

(56) References cited:
- WO-A1-2012/096706
- US-A1- 2008 140 092
- US-A1- 2012 172 924
- US-A1- 2012 184 972

## Description

The present invention relates to a suture achor delivery system comprising a control mechanism for prioritized deployment of two suture anchors.

### BACKGROUND OF THE INVENTION

When a soft tissue, or a portion of a tissue, such as muscle, ligament, or cartilage, tears, surgery to repair the detached soft tissue is often required. The goal of such surgery is to suture the torn portion of the tissue to thereby repair the tear and reconstitute the tissue back to its original status. Traditionally, repair was accomplished by sewing the tissue together with two needles and a suture, then tying knots to secure the suture within the tissue. To simplify the wound closure procedure and to improve fixation, various types of suture anchors have been developed, such as those described in U.S. Pat. No. 7,153,312 B1 to Torrie et al. and U.S. Pat. No. 6,972,027 B2 to Fallin et al.

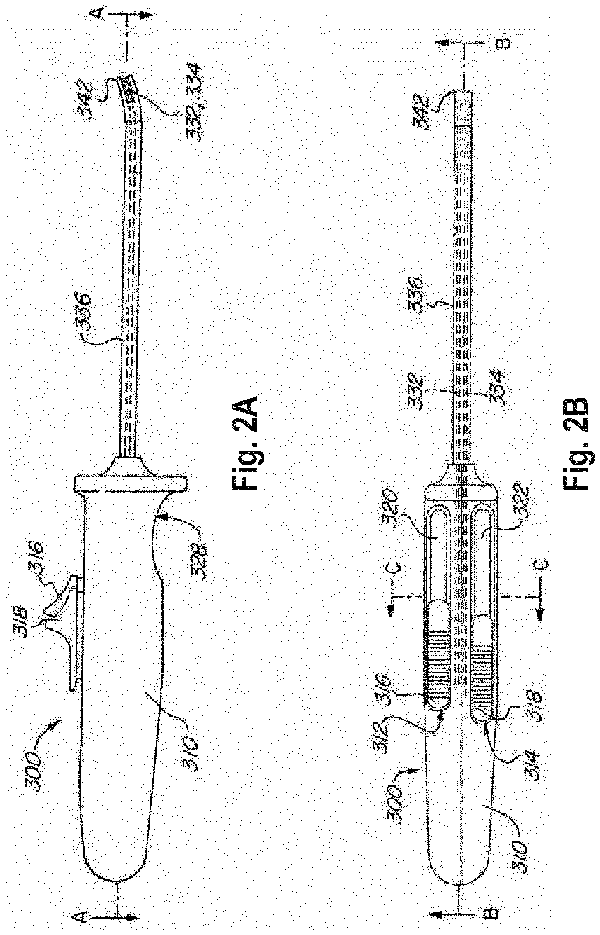

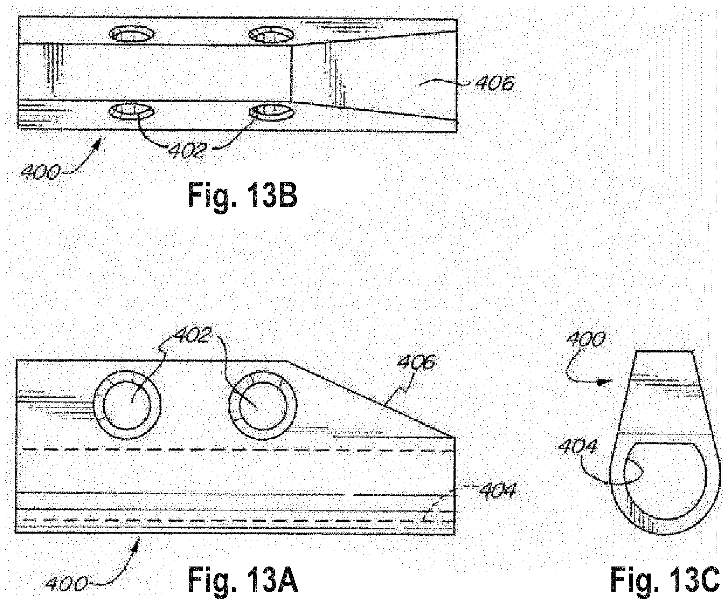

Torrie et al. disclose a closure device for repairing a tear in soft tissue comprising a suture coupled with two fixation members. Each fixation member comprises two holes through which the suture is received. The suture is immovably fixed to the first fixation member, but is freely movable relative to the second fixation member. Therefore, a retaining element, in the form of a slip knot or overhand knot, must be provided on the free end of the suture to prevent the suture from loosening between the fixation members when a tension is applied. When an overhand knot is used, the surgeon must use a knot pusher in order to shorten the length of suture between the fixation members and close the tear. As illustrated in Figures 2A-2I and 13-13B, the knots required by this system are particularly complicated to tie and correctly position.

Fallin et al. disclose a suture anchor delivery system comprising two suture anchors secured together by a suture. Similar to Torrie et al., the suture is immovably fixed to the first fixation member. The suture is received in the second fixation member such that pulling on the loose end of the suture causes it to selectively lock to the second anchor. Once the fixation members are implanted, tightening the portion of the suture between them requires a highly coordinated procedure. The surgeon must simultaneously pull back on both free ends of a retraction line and the free end of the suture to cause the suture to unlock from the second fixation device. Then, while continuing to pull back on the free end of the suture, the surgeon must slowly release the retraction line at a complementary rate. If necessary, this process is repeated until all of the slack is removed from between the anchors.

Unfortunately, the devices of Torrie et al. and Fallin et al. are unsatisfactory for a variety of reasons. What is desired, therefore, is a suture holding system for use in the repair of soft tissue tears that does not require the use of knots, knot pushers, and retraction lines in order to implant and utilize the devices.

Several devices are also known for the delivery of such suture anchors. Both Fallin et al. and Torrie et al. disclose delivery devices in which two or more suture anchors are delivered via a single needle and single pusher mechanism. Such devices provide the surgeon with little freedom for individually deploying the suture anchors and make it difficult to make adjustments once deployment of the first anchor has begun. As a result, delivery devices which allow for the independent delivery of at least two suture anchors have been developed. For example, U.S. Patent No. 7,905,904 to Stone et al. discloses a delivery device having separate needles and pushers for delivering each of two implants. However, this device undesirably has the pusher mechanisms extending from opposing surfaces of the body of the device. Further, this device does not provide any means for the surgeon to rigidly fix the position of the delivery needle, which would offer more flexibility in the deployment process. What is desired, therefore, is a suture holding system for use in the repair of soft tissue tears with a delivery device that allows for independent and prioritized deployment of at least two suture anchors that also allows a surgeon to fix one or more of the driver mechanisms in at least one position.

US 2008/140092 A1 discloses a suture anchor delivery system according to the preamble of appended independent claim 1.

### SUMMARY OF THE INVENTION

The invention is defined by the appended independent claim 1. A preferred embodiment is defined by the dependend claim.

### BRIEF DESCRIPTION OF THE DRAWINGS

FIG. 1 is a projected view of an embodiment of the delivery device of the present invention.
FIG. 2A is a side view of an embodiment of the delivery device shown in FIG. 1.
FIG. 2B is a top view of an embodiment of the delivery device shown in FIG. 1.
FIG. 3A is a sectional view of an embodiment of the delivery device shown in FIG. 2A taken along the line A-A.
FIG. 3B is a sectional view of an embodiment of the delivery device shown in FIG. 2B taken along the line B-B.
FIG. 4 is a sectional view of an embodiment of the delivery device shown in FIG. 2B taken along the line C-C.
FIG. 5 is a side view of an embodiment of the driver mechanism for use in the delivery device of FIG. 1.
FIG. 6A is a side view of an embodiment of the delivery device shown in FIG. 1.
FIG. 6B is a top view of an embodiment of the delivery device shown in FIG. 1.
FIG. 7A is a sectional view of an embodiment of the delivery device shown in FIG. 6A taken along the line D-D.
FIG. 7B is a sectional view of an embodiment of the delivery device shown in FIG. 6B taken along the line E-E.
FIG. 8 is a sectional view of an embodiment of the delivery device shown in FIG. 6B taken along the line F-F.
FIG. 9A is a side view of an embodiment of the delivery device shown in FIG. 1.
FIG. 9B is a top view of an embodiment of the delivery device shown in FIG. 1.
FIG. 10A is a sectional view of an embodiment of the delivery device shown in FIG. 9A taken along the line G-G.
FIG. 10B is a sectional view of an embodiment of the delivery device shown in FIG. 9B taken along the line H-H.
FIG. 11 is a sectional view of an embodiment of the delivery device shown in FIG. 9B taken along the line I-I.
FIG. 12 is a projected view of an embodiment of a suture anchor for use with the delivery device depicted in Fig. 1.
FIG. 13A is a side view of an embodiment of a suture anchor for use with the delivery device depicted in FIG. 1.
FIG. 13B is a top view of an embodiment of a suture anchor for use with the delivery device depicted in FIG. 1.
FIG. 13C is a back view of an embodiment of a suture anchor for use with the delivery device depicted in FIG. 1.
FIG. 14 is a top partial sectional view of an embodiment of the delivery device depicted in FIG. 1, showing two suture anchors.

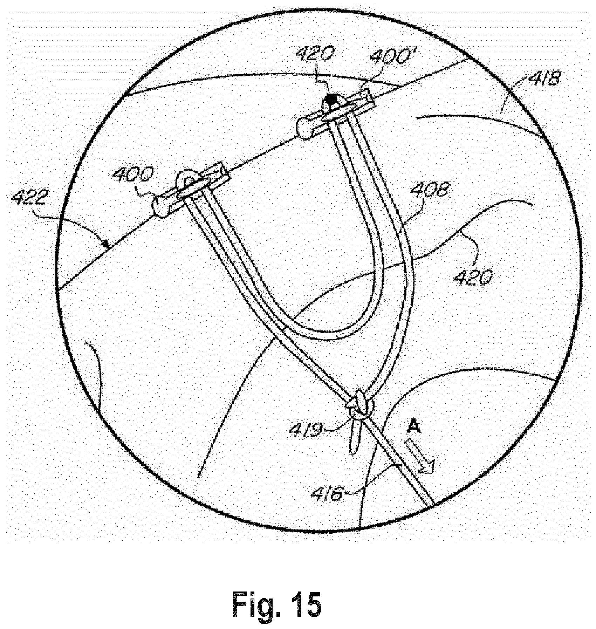

FIG. 15 is a view of an embodiment of the suture holding system having two suture anchors being used to repair a tear in soft tissue.
FIG. 16 is a projected partial view of an embodiment of the suture holding system, including an embodiment of the delivery device depicted in FIG. 1.

### DETAILED DESCRIPTION OF THE INVENTION

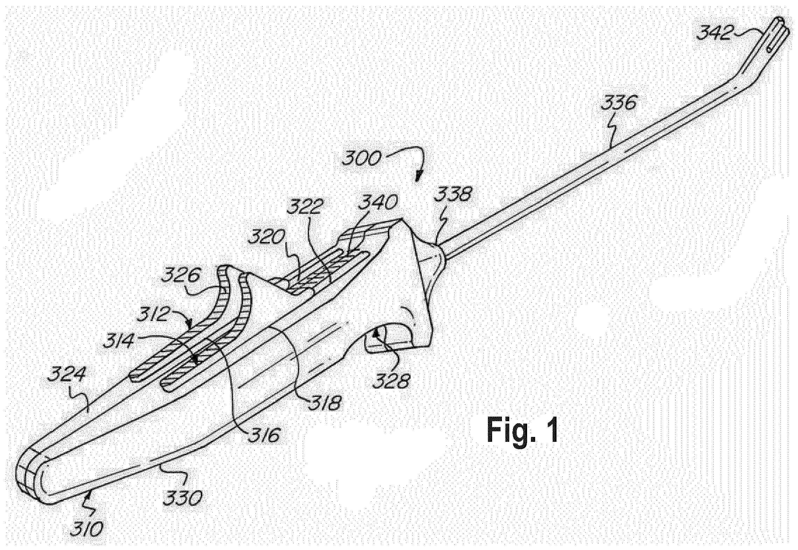

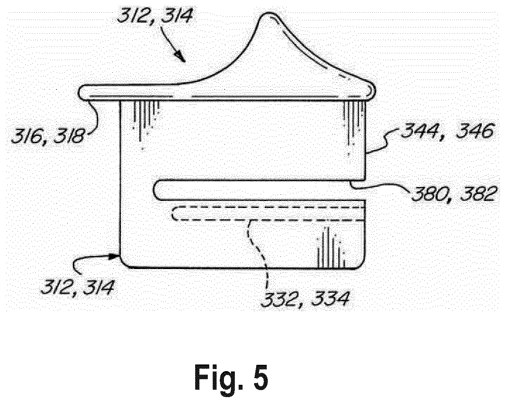

An embodiment of a delivery device 300, according to the present invention, is shown in Figure 1. The delivery device 300 includes a housing 310, which may be formed in two separate pieces and connected together or may be formed as one continuous piece, with first 312 and second 314 driver mechanisms coupled thereto for independently deploying two suture anchors (not shown). The driver mechanisms 312, 314 are slidably disposed in first 320 and second 322 channels open to the top surface 324 of the housing 310 and include first 316 and second 318 pushers. Grips 326 may be provided on the pushers 316, 318 to prevent the surgeon's fingers or thumb from slipping during deployment. A finger hold 328 may also be provided on the bottom surface 330 of the housing 310. As shown in detail in Figure 5, first 312 and second 314 driver mechanisms also include first 344 and second 346 fins, respectively, extending downwardly from the pushers 316, 318. The fins 344, 346 are slidably received within channels 320, 322.

First 332 and second 334 delivery needles for carrying the two suture anchors are coupled to the driver mechanisms 312 and 314 by conventional means and extend through a cannula 336 which may be provided at a distal end 338 of the housing 310. The distal end 342 of the cannula 336 may be forked to allow for passage of a suture connecting the two anchors. Additionally, the distal end of the cannula 342 and the distal end of the needles 332, 334 may have a slight curvature to aid in implanting to the suture anchors. Calibration marks 340, which determine the penetration depth of needle, may be provided on the top surface 324 of the housing for assisting the surgeon.

The improved delivery device 300 also includes a control mechanism 348 for controlling the sequence of deployment of the driver mechanisms 312, 314. Specifically, the control mechanism 348 ensures that the first driver mechanism 312 is deployed before the second driver mechanism 314, such that the first suture anchor is implanted before the second suture anchor. This sequence of implantation is important for maintaining the orientation of the first suture anchor with respect to the second suture anchor so that the suture threaded between the two anchors does not become tangled, twisted or reversed, which will make tightening of the suture and repair of the torn tissue difficult, if not impossible.

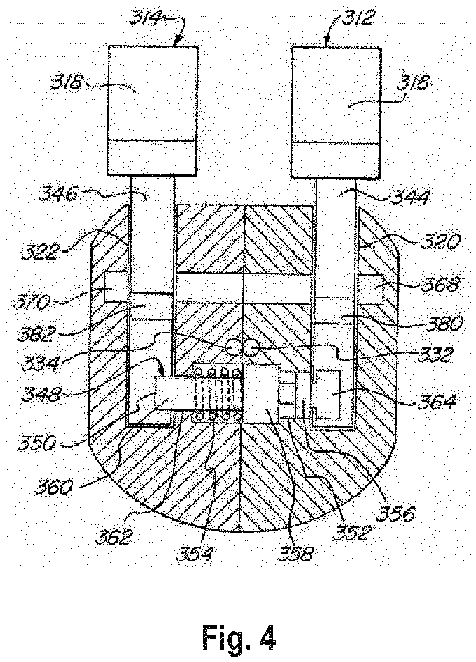

Generally, the control mechanism 348 does not allow the second driver mechanism 314 to be advanced in a distal direction until the first driver mechanism 312 has been advanced to a fully extended position and the first suture anchor is implanted. Figures 2A, 2B, 3A, 3B and 4 illustrate both driver mechanisms 312, 314 in a fully retracted position. In this position, needles 332, 334 are concealed within the cannula 336.

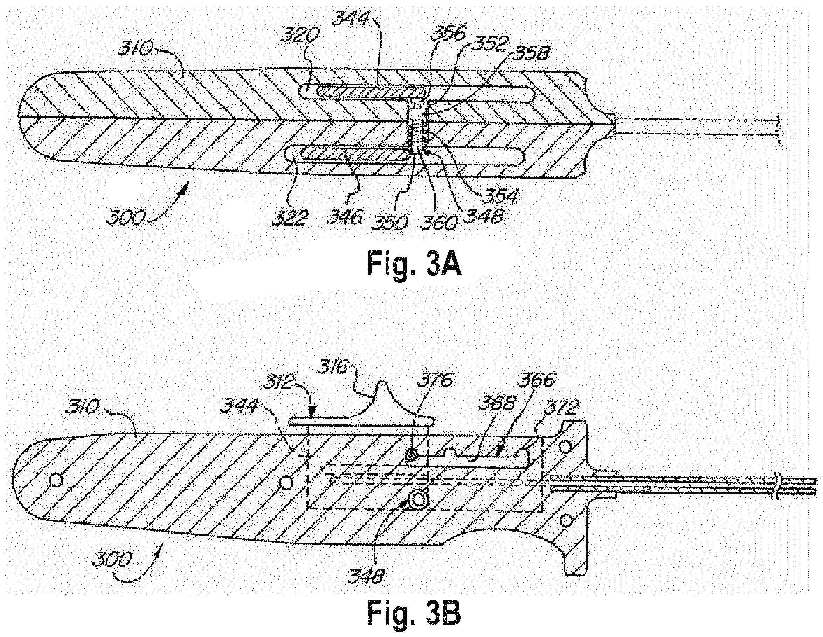

In one embodiment, the control mechanism 348 includes a pin 350 slidably disposed in a lateral bore 352 in the housing 310. As shown in Figure 3A, when the first driver mechanism 312 is fully retracted, spring 354 keeps the head 356 of pin 350 biased towards the fin 344 of the first driver mechanism. In this position, one end of the pin 350 lies distal of the fin 346 of the second driver mechanism 314, preventing it from moving in a distal direction. The pin 350 also includes a stop 358, having a diameter conforming approximately to the diameter of the bore 352 and is larger than the diameter of the body portion 360 of the pin.

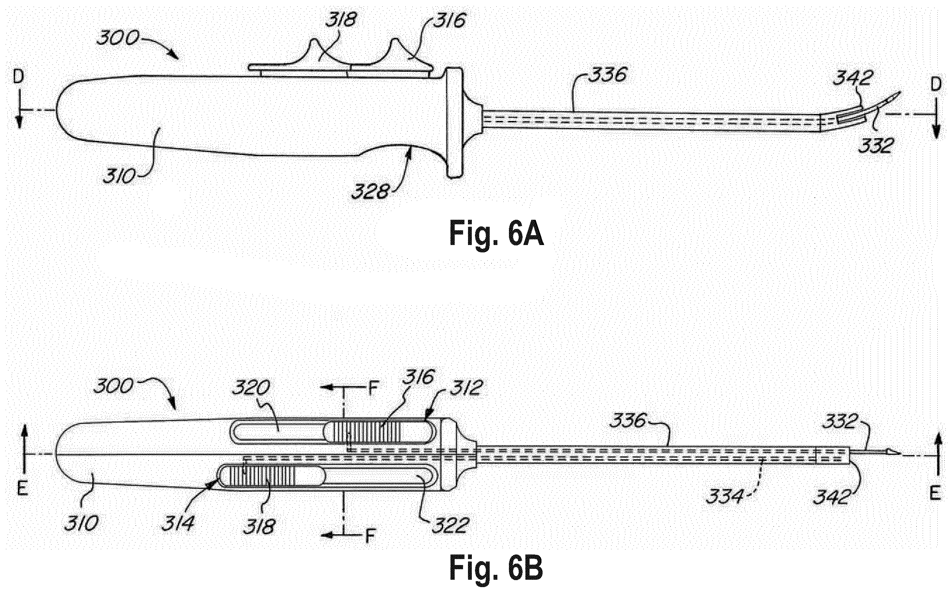

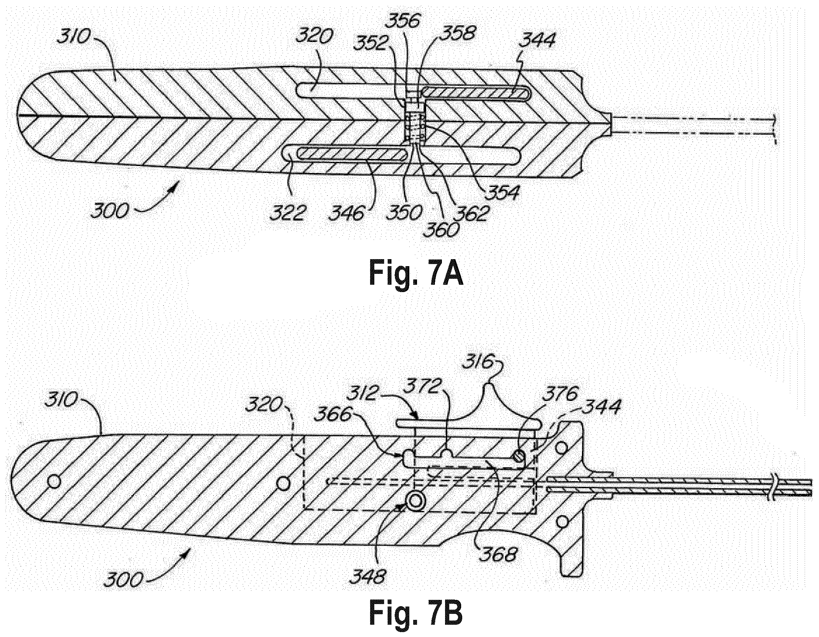

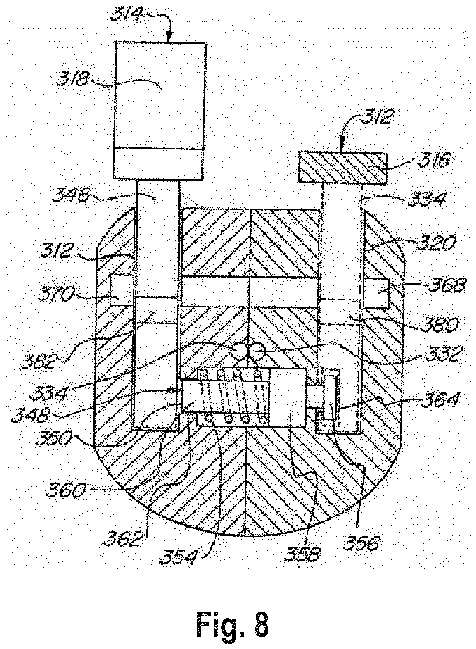

Figures 6A, 6B, 7A, 7B and 8 illustrate the first driver mechanism 312 in a fully extended position, while the second driver mechanism 314 remains in a fully retracted position. Until the first driver mechanism 312 is moved into this position, the second driver mechanism 314 cannot be advanced. As shown in Figure 7A, when the first driver mechanism 312 is fully advanced, the fin 344 is moved completely distal of the pin head 356, allowing clearance from the lateral bore 352, into the channel 320. Spring 354, having been compressed in the locked position of the control mechanism 348, now causes the pin to move laterally into the channel 320. As shown in Figure 8, a track 364, which runs the length of fin 344, is also provided. When the surgeon chooses to return the first driver mechanism 312 from the fully extended to the fully retracted position, the head 356 will pass through track 364. Once pin 350 has moved laterally into channel 320, the second end of the pin no longer blocks second driver mechanism 314 from moving longitudinally in channel 312 and the surgeon may implant the second suture anchor.

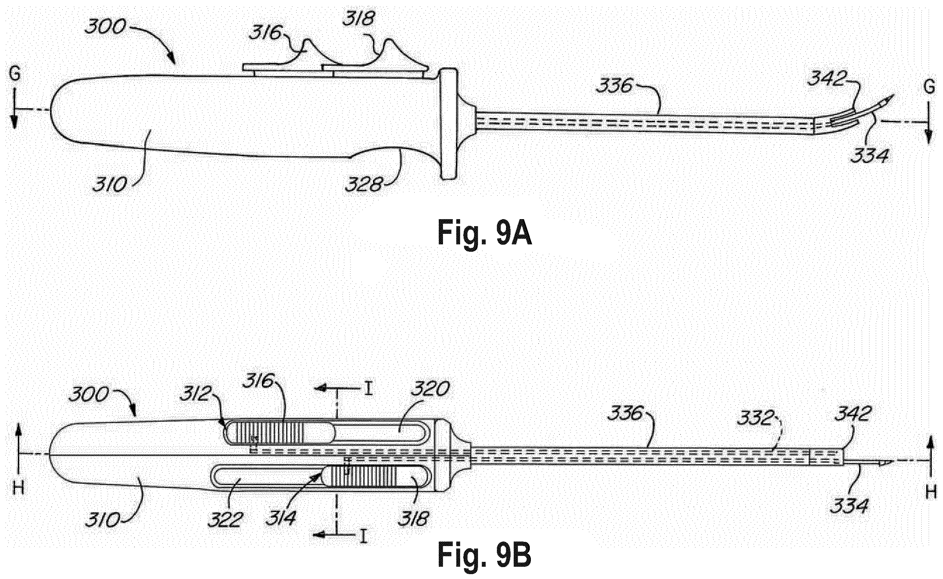

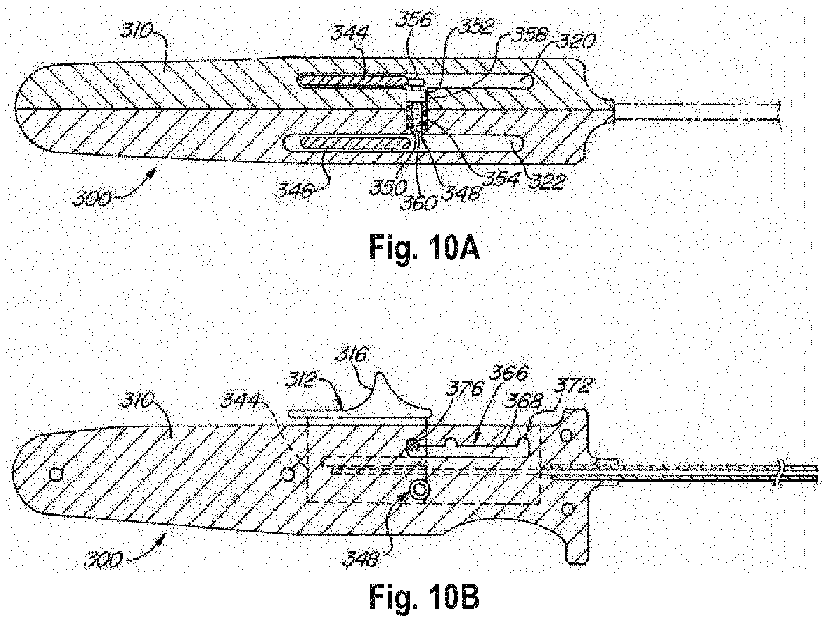

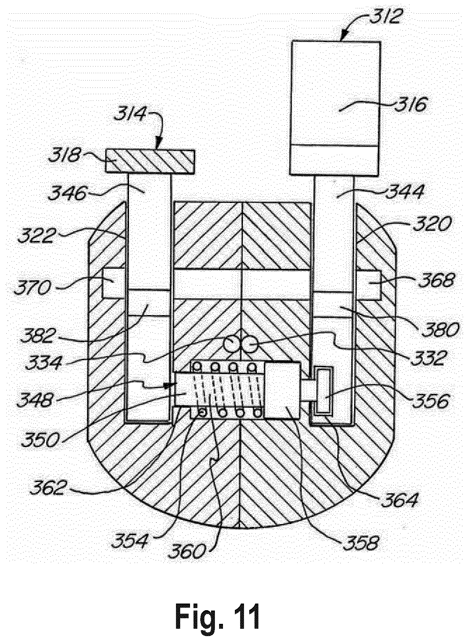

In Figures 9A, 9B, 10A, 10B and 11, first driver mechanism 312 has been returned to a fully retracted state and the second driver mechanism 314 is advanced to a fully extended state, thus allowing the surgeon to implant the second suture anchor.

The delivery device 300 may also be provided with a toggle mechanism 366. It is often desirable for the surgeon to fix or lock a driver mechanism, and thus a delivery needle, in a particular position during the implantation procedure. For example, the surgeon may wish to fix the driver mechanism in a fully extended position, or in an intermediate position in between the fully retracted and fully extended positions. This ability to fix the driver mechanism provides additional rigidity to the delivery device in that the surgeon need not physically hold the pusher in the desired position. Further, the ability to fix the driver mechanism in an intermediate position, where the delivery needle is not fully extended, provides the surgeon with the flexibility to change the position of the needle within the tissue before the suture anchor is implanted. For safety and sterility reasons, it may also be desirable to fix the driver mechanisms in a fully retracted position so that they are not accidentally advanced prior to the surgical procedure, such as during packaging or shipping, or after the surgical procedure has been completed.

As shown in Figure 3B, the toggle mechanism 366 includes at least one toggle channel 368, 370 in the housing and at least one detent 372, 374 positioned along and opening up to the channel 368, 370. The detents 372, 374 define the positions in which the surgeon may fix a driver mechanism. In one embodiment, the toggle mechanism 366 includes three detents 372, one defining a fully retracted position, a fully extended position and an intermediate position lying therebetween. To lock the driver mechanism in one of the preset positions, pin 376, 378, connected to the fin 344, 346, is received in the toggle 372, 374 of the desired position. To achieve this, a slit 380, 382 is provided in the fin 344, 346 which allows the pin 376, 378 to essentially be squeezed together and pressed downward from a detent and into toggle channel 368, 370. The toggle mechanism 366 may be provided in one or both of the driver mechanisms 312, 314.

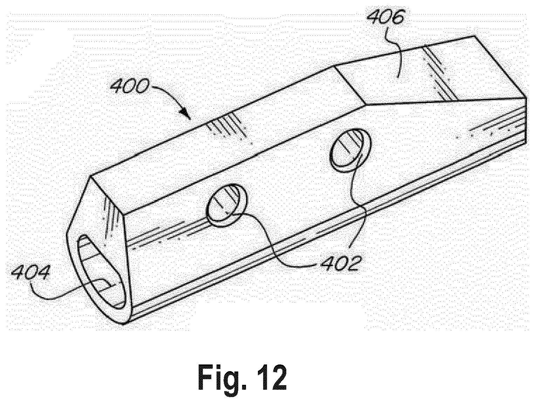

One embodiment of a suture anchor 400 is shown in Figure 12. The anchor 400 includes at least two eyelets 402 and a longitudinal channel 404 for receiving the distal end of a delivery needle 332, 334. The channel 404 and at least a distal portion of the needle 332, 334 may be non-circular in cross section to prevent the anchor 400 from rotating on the needle. The anchor 400 may also be provided with a tapered leading edge 406 to facilitate in implantation.

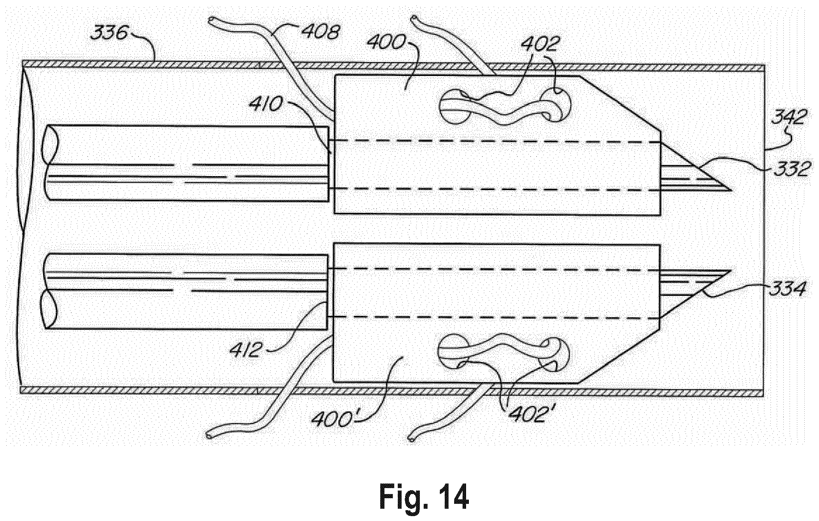

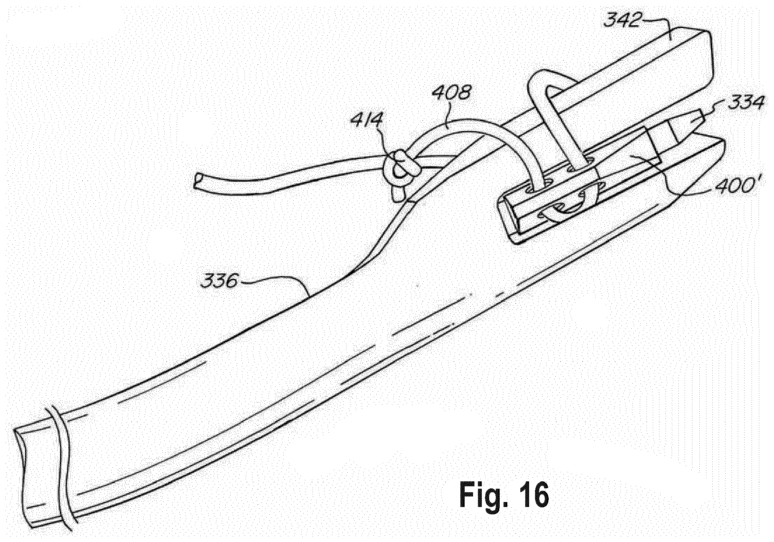

In operation, first and second anchors 400, 400', with a suture 408 threaded therebetween, are loaded on to needles 332, 334 engaging shoulders 410, 412 as shown in Figure 14. In one embodiment, a single strand of suture 408 is threaded through the first anchor 400 and then through the second anchor 400', with a locking slip-knot 414 connecting the two ends of suture. The free end 416 of suture 408 may be pulled in the direction of arrow A to tighten the loop of suture passing through the tissue 418 to close tear 420. Any other suitable knot may also be used. The suture, loaded on the anchors, may then pass out of the distal end 342 of the cannula 336 through the slots therein, as shown in Figure 16.

To implant the anchors 400, 400', the surgeon positions the delivery device 300 at the area of interest, for example, near a tear in a meniscus. The surgeon then engages the first driver mechanism 312, for example, by placing his/her thumb on the first pusher 316, and advances the first driver mechanism 312 in a distal direction thereby advancing first needle 332 carrying the first implant 400. As the surgeon advances the first driver mechanism 312, the first needle 332, which may have a pointed distal end, then enters tissue 418, crosses the tear 420, and continues until the needle 332 exits the surface 422 of the tissue. In the interim of this process, the surgeon may use the toggle mechanism 366 to fix the driver mechanism 312 at an intermediate position to, for example, determine if the needle is properly positioned across the tear. If the surgeon does not like the positioning, he may retract the needle and correct the placement.

Once the driver mechanism reaches its fully extended position, the first anchor 400 will be disengaged from the first needle. The surgeon may also fix the first needle 332 in the fully extended position via the toggle mechanism 366 while he/she, for example, disengages the first anchor 400 or adjusts the suture 408. As described above, until the first driver mechanism 312 reaches the fully extended position, the control mechanism 348 prevents the second driver mechanism 314 from advancing in a distal direction. The same procedure is used to implant the second suture anchor 400'. Once both anchors 400, 400' are implanted, the surgeon may then pull free end 416 of the suture in the direction of arrow A to tighten the length of suture between the implants and close the tear 420.

Additionally, in one embodiment, a stop knot 420, which may be a single overhand knot, may also be provided in the length of suture between the eyelets 402' of the second suture anchor 400'. This stop knot 420 aids in tightening of the anchor construct. Without the knot, tightening of the construct can be difficult because of the multiple lengths of suture that pass through the tissue 418, which can cause the suture to get caught up and not slide well. With the stop knot 420, the length of the suture portion between the slip-knot 414 and stop knot 420 is fixed and not adjustable.

## Claims

1. A suture anchor delivery system comprising:
a housing (310) having a distal end and a proximal end, a first longitudinal channel (320), and a second longitudinal channel (322) substantially parallel to the first longitudinal channel; a first driver mechanism (312) slidably disposed in the first longitudinal channel, and a second driver mechanism (314) slidably disposed in the second longitudinal channel, such that each driver mechanism is movable in a longitudinal direction with respect to said housing (310);
a first delivery needle (332) and a second delivery needle (334), said first delivery needle (332) connected at its proximal end to said first driver mechanism (312) and said second delivery needle (334) connected at its proximal end to said second driver mechanism (314);
said first driver mechanism (312) and said second driver mechanism (314) each having fully retracted and fully extended longitudinal positions; and
a control mechanism (348) operable to prevent said second driver mechanism (314) from being moved in a distal direction until said first driver mechanism (312) is longitudinally advanced to said fully extended position, **characterized in that**
the housing further comprises a lateral bore (352) in between, and opening into, the first and the second longitudinal channel, and **in that** the control mechanism (348) comprises a pin (350) having a head (356) and a body (360), said pin (350) being slidably disposed in the lateral bore such that the pin is movable in a lateral direction with respect to said housing (310), and
a spring (354) received on the pin body (360) between the head and the housing, biasing the pin towards the first driver mechanism, the control mechanism (348) having a locked position in
which the first driver mechanism (312) is in a longitudinally retracted position, wherein the spring (354) biases the pin head (356) laterally against the first driver mechanism (312), and
in which at least a portion of the pin body (360) is positioned in the second longitudinal channel, distal of said second driver mechanism (314), thus preventing the second driver mechanism (314) from being longitudinally advanced in the second longitudinal channel in a distal direction,
the control mechanism (348) further having an unlocked position
in which the first driver mechanism (312) is in its fully extended longitudinal position, wherein the spring biases the pin head (356) laterally into the first longitudinal channel into a position that is proximal to the first driver mechanism (312),
so that the pin body does no longer prevent the second driver mechanism (314) from being longitudinally advanced in the second longitudinal channel in a distal direction.

2. The suture anchor delivery system of claim 1, wherein said control mechanism (348) is in a locked position until said first driver mechanism (312) is longitudinally advanced to said fully extended position and in an unlocked position after said first driver mechanism (312) is longitudinally advanced to said fully extended position.

## Patentansprüche

1. Ein Fadenankerzuführsystem, umfassend:
ein Gehäuse (310) mit einem distalen Ende und einem proximalen Ende, einem ersten Längskanal (320) und einem zweiten Längskanal (322), der im Wesentlichen parallel zum ersten Längskanal verläuft;
einen ersten Mitnehmermechanismus (312), der in dem ersten Längskanal verschiebbar angeordnet ist, und einen zweiten Mitnehmermechanismus (314), der in dem zweiten Längskanal verschiebbar angeordnet ist, derart, dass jeder Mitnehmermechanismus in einer Längsrichtung in Bezug auf das Gehäuse (310) beweglich ist;
eine erste Zuführnadel (332) und eine zweite Zuführnadel (334), wobei die erste Zuführnadel (332) an ihrem proximalen Ende mit dem ersten Mitnehmermechanismus (312) und die zweite Zuführnadel (334) an ihrem proximalen Ende mit dem zweiten Mitnehmermechanismus (314) verbunden ist;
wobei der erste Mitnehmermechanismus (312) und der zweite Mitnehmermechanismus (314) jeweils vollständig eingefahrene und vollständig ausgefahrene Längspositionen aufweisen; und
einen Steuermechanismus (348), der derart betreibbar ist, dass verhindert wird, dass der zweite Mitnehmermechanismus (314) in eine distale Richtung bewegt wird, bis der erste Mitnehmermechanismus (312) in Längsrichtung zu der vollständig ausgefahrenen Position vorgeschoben wird, **dadurch gekennzeichnet, dass**
das Gehäuse ferner zwischen dem ersten und dem zweiten Längskanal eine seitliche Bohrung (352) aufweist, die in diesen mündet, und dass
der Steuermechanismus (348) einen Stift (350) mit einem Kopf (356) und einem Körper (360) aufweist, wobei der Stift (350) derart in der seitlichen Bohrung verschiebbar angeordnet ist, dass der Stift in einer seitlichen Richtung in Bezug auf das Gehäuse (310) beweglich ist, und
eine Feder (354), die auf dem Stiftkörper (360) zwischen dem Kopf und dem Gehäuse aufgenommen ist, die den Stift in Richtung des ersten Mitnehmermechanismus vorspannt,
wobei der Steuermechanismus (348) eine verriegelte Position aufweist,
in der sich der erste Mitnehmermechanismus (312) in einer in Längsrichtung zurückgezogenen Position befindet, wobei die Feder (354) den Kopf des Stiftes (356) seitlich gegen den ersten Mitnehmermechanismus (312) vorspannt, und
in der zumindest ein Teil des Stiftkörpers (360) in dem zweiten Längskanal angeordnet ist, distal des zweiten Mitnehmermechanismus (314), wodurch verhindert wird, dass der zweite Mitnehmermechanismus (314) in Längsrichtung im zweiten Längskanal in einer distalen Richtung vorwärts bewegt wird, und
wobei der Steuermechanismus (348) ferner eine entriegelte Position aufweist, in der sich der erste Mitnehmermechanismus (312) in seiner vollständig ausgefahrenen Längsposition befindet,
wobei die Feder den Kopf des Stiftes (356) seitlich in den ersten Längskanal in eine Position vorspannt, die proximal zum ersten Mitnehmermechanismus (312) ist,
so dass der Stiftkörper den zweiten Mitnehmermechanismus (314) nicht mehr daran hindert, in einer distalen Richtung im zweiten Längskanal in Längsrichtung vorzurücken.

2. Fadenankerzuführsystem nach Anspruch 1, wobei sich der Steuermechanismus (348) in einer verriegelten Position befindet, bis der erste Mitnehmermechanismus (312) in Längsrichtung zu der vollständig ausgefahrenen Position vorgerückt ist, und in einer entriegelten Position ist, nachdem der erste Mitnehmermechanismus (312) in Längsrichtung zu der vollständig ausgefahrenen Position vorgerückt ist.

## Revendications

1. Système de distribution d'ancre de suture, comprenant:
un boîtier (310) présentant une extrémité distale et une extrémité proximale, un premier canal longitudinal (320) et un second canal longitudinal (322) sensiblement parallèle au premier canal longitudinal;
un premier mécanisme d'entraînement (312) disposé de façon coulissante dans le premier canal longitudinal, et un second mécanisme d'entraînement (314) disposé de façon coulissante dans le second canal longitudinal, de telle sorte que chaque mécanisme d'entraînement soit déplaçable dans une direction longitudinale par rapport audit boîtier (310);
une première aiguille de distribution (332) et une seconde aiguille de distribution (334), ladite première aiguille de distribution (332) étant connectée à son extrémité proximale audit premier mécanisme d'entraînement (312), et ladite seconde aiguille de distribution (334) étant connectée à son extrémité proximale audit second mécanisme d'entraînement (314);
ledit premier mécanisme d'entraînement (312) et ledit second mécanisme d'entraînement (314) présentant chacun des positions longitudinales complètement rétractée et complètement étendue; et
un mécanisme de commande (348) actionnable pour empêcher ledit second mécanisme d'entraînement (314) d'être déplacé dans une direction distale jusqu'à ce que ledit premier mécanisme d'entraînement (312) ait avancé de façon longitudinale jusqu'à ladite position complètement étendue,
**caractérisé en ce que** le boîtier comporte en outre un alésage latéral (352) entre et s'ouvrant dans le premier et le second canaux longitudinaux,
et **en ce que** le mécanisme de commande (348) comprend une broche (350) présentant une tête (356) et un corps (360), ladite broche (350) étant disposée de façon coulissante dans l'alésage latéral de telle sorte que la broche soit déplaçable dans une direction latérale par rapport audit boîtier (310), et
un ressort (354) reçu sur le corps de broche (360) entre la tête et le boîtier, poussant la broche en direction du premier mécanisme d'entraînement,
le mécanisme de commande (348) présentant une position verrouillée, dans laquelle le premier mécanisme d'entraînement (312) se trouve dans une position longitudinalement rétractée, dans laquelle le ressort (354) pousse la tête de broche (356) latéralement contre le premier mécanisme d'entraînement (312), et
dans lequel au moins une partie du corps de broche (360) est positionnée dans le second canal longitudinal, à distance dudit second mécanisme d'entraînement (314), empêchant de ce fait le second mécanisme d'entraînement (314) d'être avancé de façon longitudinale dans le second canal longitudinal dans une direction distale,
le mécanisme de commande (348) présentant en outre une position déverrouillée, dans laquelle le premier mécanisme d'entraînement (312) se trouve dans sa position longitudinale complètement étendue, dans laquelle le ressort pousse la tête de broche (356) latéralement dans le premier canal longitudinal dans une position qui est proche du premier mécanisme d'entraînement (312),
de telle sorte que le corps de broche n'empêche plus le second mécanisme d'entraînement (314) d'être avancé de façon longitudinale dans le second canal longitudinal dans une direction distale.

2. Système de distribution d'ancre de suture selon la revendication 1, dans lequel ledit mécanisme de commande (348) se trouve dans une position verrouillée jusqu'à ce que ledit premier mécanisme d'entraînement (312) ait avancé de façon longitudinale jusqu'à ladite position complètement étendue et se trouve dans une position déverrouillée après que ledit premier mécanisme d'entraînement (312) ait avancé de façon longitudinale jusqu'à ladite position complètement étendue.
